(19) Europäisches Patentamt
European Patent Office
Office européen des brevets

(11) EP 3 998 333 A1

(12) EUROPEAN PATENT APPLICATION
published in accordance with Art. 153(4) EPC

(43) Date of publication:
18.05.2022 Bulletin 2022/20

(21) Application number: 20836408.3

(22) Date of filing: 10.07.2020

(51) International Patent Classification (IPC):
*C12N 5/071* (2010.01) *C12N 5/10* (2006.01)

(52) Cooperative Patent Classification (CPC):
C12N 5/10

(86) International application number:
PCT/JP2020/027111

(87) International publication number:
WO 2021/006346 (14.01.2021 Gazette 2021/02)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 10.07.2019 JP 2019128526

(71) Applicant: Osaka University
Suita-shi, Osaka 565-0871 (JP)

(72) Inventors:
- KINOOKA, Masahiro
Suita-shi, Osaka 565-0871 (JP)
- KIM, Meehae
Suita-shi, Osaka 565-0871 (JP)

(74) Representative: Dehns
St. Bride's House
10 Salisbury Square
London EC4Y 8JD (GB)

# (54) METHOD FOR PROMOTING CELL PROLIFERATION, AND METHOD FOR PREPARING CELL CLUSTER

(57) Provided is a method that is able to simply promote cell proliferation.

An aspect relates to a method for promoting proliferation of cells. The method includes adding a precursor of AGEs to a culture medium containing a cell aggregate, and culturing the cell aggregate in the culture medium to which the precursor of AGEs has been added

EP 3 998 333 A1

**Description**

Technical Field

The present disclosure relates to a method for promoting cell proliferation, a method for preparing cell aggregates, cell aggregates obtained by the method, an anti-fibrillating agent for cell aggregates, and a kit for culturing cell aggregates.

Background Art

Stem cells have the ability to differentiate into various cell types (pluripotency) and the ability to self-renew. Stem cells are classified into two types: tissue stem cells used to maintain and repair the tissue; and pluripotent stem cells such as embryonic stem cells (ES cells) and induced pluripotent stem cells (iPS cells).

Stem cells are expected to be applied to regenerative medicine in recent years because of their pluripotency and self-renewal ability. Therefore, there is a need for technology that allows stem cells to proliferate simply and efficiently.

Prior Art Documents

Patent Documents

Patent Document 1: WO 2015/033558

Disclosure of Invention

Problem to be Solved by the Invention

One aspect of the present disclosure provides a method that is able to simply promote cell proliferation in cell aggregates of, e.g., stem cells.

Means for Solving Problem

One aspect of the present disclosure relates to a method for promoting proliferation of cells. The method includes adding a precursor of advanced glycation end products (AGEs) to a culture medium containing a cell aggregate, and culturing the cell aggregate in the culture medium to which the precursor of AGEs has been added.

Another aspect of the present disclosure relates to a method for preparing a cell aggregate. The method includes adding a precursor of AGEs to a culture medium containing a cell aggregate, and culturing the cell aggregate in the culture medium to which the precursor of AGEs has been added.

Another aspect of the present disclosure relates to a method for unraveling an extracellular matrix shell (ECM shell) of a cell aggregate. The method includes culturing cells to form a cell aggregate, and adding a precursor of AGEs to a culture medium containing the cell aggregate.

Another aspect of the present disclosure relates to a cell aggregate obtained by the above methods.

Another aspect of the present disclosure relates to an anti-fibrillating agent for a cell aggregate. The anti-fibrillating agent contains a precursor of AGEs.

Another aspect of the present disclosure relates to a kit for culturing a cell aggregate. The kit contains a precursor of AGEs and a Rho-kinase (Rho-associated coiled-coil containing protein kinase: ROCK) inhibitor.

Effects of the Invention

In one aspect, the present disclosure can simply promote cell proliferation in cell aggregates of, e.g., stem cells.

Brief Description of Drawings

[FIG. 1A] FIG. 1A is a series of photomicrographs showing an example of the results of Test Example 1: photomicrographs of iPS cell aggregates before the addition of methylglyoxal (MG) (Day 4) and after the addition of MG (Day 5, 6, 7).

[FIG. 1B] FIG. 1B is a series of photomicrographs showing an example of the results of Test Example 1: photomicrographs of iPS cell aggregates before the addition of MG (Day 4) and after the addition of MG (Day 5,6, 7).

[FIG. 2] FIG. 2 illustrates a scheme in Test Example 2.

[FIG. 3] FIG. 3 illustrates a scheme in Test Example 3.

[FIG. 4] FIG. 4 is a series of photomicrographs showing an example of the results of Test Examples 2 and 3.

[FIG. 5] FIG. 5 is a graph showing an example of the results of Test Example 4. The graph represents the relationship between the concentration of MG added and the cell proliferation rate.

[FIG. 6] FIG. 6 is a series of photomicrographs showing an example of the results of Test Examples 5 and 6.

[FIG. 7] FIG. 7 is a series of photomicrographs showing an example of the results of Test Example 7.

[FIG. 8] FIG. 8 is a graph showing an example of the results of Test Example 8. The graph represents the relationship between the concentration of MG added and the cell proliferation rate.

Description of the Invention

In aggregate culture of stem cells such as mesenchymal stem cells, iPS cells, and ES cells and other cells, when cell aggregates of the cells are cultured for a certain period of time, the proliferation of cells forming the aggregates may be stopped or the proliferation rate may be reduced (causing a decrease in proliferative capacity). The present disclosure is based on the finding that the decreased proliferative capacity can be restored by bringing a precursor of AGEs such as methylglyoxal into contact with the cell aggregates.

Moreover, the present disclosure is based on the finding that the proliferative capacity of cells in the cell aggregates can be further improved by bringing a ROCK inhibitor into contact with the cell aggregates that have been in contact with the precursor of AGEs such as methylglyoxal.

The details of the mechanism of restoration of the proliferative capacity of cells in the cell aggregates due to the contact between the cell aggregates and the precursor of AGEs such as methylglyoxal are not fully clear, but can be assumed as follows.

When cell aggregates of, e.g., stem cells are cultured for a certain period of time, the proliferation may be stopped or the proliferation rate may be reduced The reason for this is considered as follows. The ECM such as collagen on the surface of cell aggregates becomes fibrillated to form a hard shell (ECM shell structure) on the surface of the cell aggregates. Consequently, the migration of cells in the cell aggregates is reduced, and the cells, in particular the cells in the aggregate center have low proliferative capacity. In other words, the formation of the ECM shell structure on the surface of the cell aggregates reduces the migration of cells in the cell aggregates, resulting in contact inhibition. This may impair the cell proliferation and/or the proliferation rate in the cell aggregates. In contrast, the addition of the precursor of AGEs such as methylglyoxal to a culture medium of the cell aggregates allows the fibrillar ECM (ECM shell) to be non-fibrillated, so that the ECM shell covering the surface of the cell aggregates is unraveled, thereby improving the migration of cells in the cell aggregates. As a result, the proliferative capacity of cells in the cell aggregates can be restored

Moreover, when the cell aggregates that have been in contact with the precursor of AGEs come into contact with a ROCK inhibitor, it is possible not only to make the fibrillar ECM shell non-fibrillated (i.e., to bring the fibrillar ECM in the ECM shell back to a near-normal ECM and/or non-fibrotic ECM), but also to promote the production of the ECM of cells in the cell aggregates. Thus, the proliferative capacity of cells in the cell aggregates can be further improved.

It should be noted that the present disclosure is not limited to these findings and mechanism.

In one or more embodiments, the present disclosure can promote the re-proliferation of cells in cell aggregates whose proliferative capacity has been reduced and/or substantially stopped, and can also improve the proliferative capacity of the cells in the cell aggregates. Moreover, the present disclosure can activate the proliferative capacity of cells that exhibit almost no proliferative capacity when they form cell aggregates. In one or more embodiments, the present disclosure enables efficient mass culture of cells. In one or more embodiments, the present disclosure can reduce the number of subcultures in proliferation or mass production of cells, as compared to the conventional technique. Therefore, in one or more embodiments, the present disclosure can achieve a large-scale expansion of cells and protect the cells from damage caused by the dissociation of cell aggregates for subculture.

According to the present disclosure, in one or more embodiments, stem cells can proliferate while maintaining their undifferentiated state in cell aggregates.

In one or more embodiments, the present disclosure can make a fibrillar extracellular matrix covering cell aggregates or cell clusters non-fibrillated, and thus can promote the proliferation of cells forming the cell aggregates or cell clusters. In one or more embodiments, the present disclosure can achieve, e.g., the reconstitution, proliferation promotion, and functional recovery of the cell aggregates or cell clusters.

In the present disclosure, the "cells" include, e.g., stem cells and differentiated cells, in one or more embodiments.

The "stem cells" are defined as cells that have the ability to differentiate into a variety of cells and the ability to self-renew. The "differentiated cells" are defined as cells that do not have differentiation potential or cells that are differentiated from stem cells. The stem cells may be, e.g., embryonic stem cells, iPS cells, somatic stem cells, or progenitor cells. In one or more embodiments, the somatic stem cells include mesenchymal stem cells, hematopoietic

stem cells, germline stem cells, nerve stem cells, epithelial stem cells, myocardial stem cells, hepatic stem cells, gastrointestinal epithelial stem cells, and skeletal muscle cells. In one or more embodiments, the stem cells may include animal-derived stem cells. In one or more embodiments, the stem cells may include stem cells cultured in vitro (Le., cultured cells). Examples of the differentiated cells include nerve cells, myocardial cells, hepatic cells, stellate cells, and cell lines of these cells. The cells that can form cell aggregates of the present disclosure are stem cells in one embodiment.

As described above, the present disclosure can activate the proliferative capacity of cells that would be reduced or substantially stopped when the cells form aggregates. Moreover, the present disclosure can prevent a decrease in the proliferative capacity after the formation of cell aggregates. Thus, in one or more embodiments, the present disclosure is suitable for the culture or proliferation of cells that exhibit almost no proliferative capacity when they form aggregates. In one or more embodiments, the cells that exhibit almost no proliferative capacity when they form aggregates may be, e.g., mesenchymal stem cell.

In the present disclosure, the "cell aggregate" refers to the state in which a plurality of cells are gathered together (aggregated) to form a cluster. In one or more embodiments, adjacent cells in a cell aggregate may be directly associated with each other or indirectly associated with each other via an extracellular matrix or the like.

In the present disclosure, the "culture medium containing a cell aggregate" contains at least a cell aggregate formed by culturing cells and a medium component, in one or more embodiments. Thus, in one or more embodiments, the method of the present disclosure may include preparing a culture medium containing a cell aggregate. The preparation process includes seeding monodispersed cells in a culture medium before the addition of AGEs or in a culture medium that does not contain AGEs, and aggregating and culturing the seeded cells to form a cell aggregate.

In the present disclosure, the "culture" includes float culture, suspension culture, and static culture such as monolayer culture. In one or more embodiments, the culture of the present disclosure includes culturing cells in such a way that they form a cell.aggregate. In one or more embodiments, the cell aggregate may be, e.g., a spheroid or a colony. The "suspension culture" is such that, e.g., cells are suspended in a culture medium and do not substantially come into contact with the bottom surface of a culture vessel while the cells are cultured In one or more embodiments, the suspension culture may be, e.g., three-dimensional culture, which includes non-contact culture using a non-contact culture vessel and suspension culture in a bioreactor.

[Method for promoting cell proliferation]

One aspect of the present disclosure relates to a method for promoting cell proliferation (i.e., a proliferation promoting method of the present disclosure). The method includes adding a precursor of AGEs to a culture medium containing a cell aggregate, and culturing the cell aggregate in the culture medium to which the precursor of AGEs has been added.In one or more embodiments, the proliferation promoting method of the present disclosure can promote the proliferation of cells in the cell aggregate. Another aspect of the present disclosure relates to a method for increasing a diameter of a cell aggregate or a method for increasing the number of cells forming a cell aggregate. These methods include adding a precursor of AGEs to a culture medium containing a cell aggregate, and culturing the cell aggregate in the culture medium to which the precursor of AGEs has been added

In one or more embodiments, the proliferation promoting method of the present disclosure includes adding a precursor of AGEs to a culture medium containing a cell aggregate. When the precursor of AGEs is added to the culture medium containing a cell aggregate so that the cell aggregate is in contact with the precursor of AGEs, in one or more embodiments, it is possible to promote the re-proliferation of cells whose proliferative capacity has been reduced and/or substantially stopped after the cells were cultured for a certain period of time and formed the cell aggregate, and also possible to activate the proliferative capacity of these cells. Thus, in one or more embodiments, the proliferation promoting method of the present disclosure includes further culturing the cell aggregate in the culture medium containing the precursor of AGEs after the addition of the precursor of AGEs to the culture medium.

In one or more embodiments, the precursor of AGEs includes an $\alpha$-dicarbonyl compound and an $\alpha$-hydroxy aldehyde compound. In one or more embodiments, the precursor of AGEs may also be referred to as a carbonyl compound that is produced by the autoxidation of glucose and/or from glucose degradation products. In one or more embodiments, the $\alpha$-dicarbonyl compound and the $\alpha$-hydroxy aldehyde compound may be reactive aldehydes. In one or more embodiments, the $\alpha$-dicarbonylcompound and the $\alpha$-hydroxy aldehyde compound may be either synthetic compounds or natural products. In the present disclosure, the "precursor of AGEs" may also be referred to as a glycation reaction intermediate.

In one or more embodiments, the $\alpha$-dicarbonylcompound includes the following: methylglyoxal; glyoxal; 3-Deoxyglucosone; malondialdehyde; butanedione; 1,2-Cyclohexanedione; phenylglyoxal; 4-Fluorophenylglyoxal; 4-Nitrophenylglyoxal; and 4-Hydroxyphenylglyoxal. Preferred examples include methylglyoxal, glyoxal, 3-Deoxyglucosone, and malondialdehyde.

In one or more embodiments, the $\alpha$-hydroxy aldehyde compound includes the following: glyceraldehyde; glycolaldehyde; lactaldehyde; and 3-Hydroxybutyraldehyde.

In one or more embodiments, the precursor of AGEs may be added to a medium after a cell aggregate is formed by culturing cells. In one or more embodiments, the precursor of AGEs may be added to a medium containing a cell aggregate. In another embodiment, the precursor of AGEs may be added to a medium containing a cell aggregate in which cells are proliferating, i.e., the proliferation is confirmed, or to a medium containing a cell aggregate in which the proliferation can hardly be confirmed or is substantially stopped. In one or more embodiments, the present disclosure includes bringing the precursor of AGEs into contact with a cell aggregate in which the proliferation can hardly be confirmed or is substantially stopped. In the present disclosure, "the proliferation can hardly be confirmed or is substantially stopped" indicates that, e.g., the diameter of the cell aggregate is not substantially increased or the number of stem cells in the cell aggregate is not substantially increased, in one or more embodiments. In one or more embodiments, the number of times the precursor of AGEs is added may be appropriately determined by the state of proliferation of the cell aggregate. The precursor of AGEs may be added one or more times.

The concentration of the precursor of AGEs in the culture medium is not particularly limited as long as the proliferation of the cell aggregate can be promoted. The concentration of the precursor of AGEs may be appropriately determined in accordance with, e.g., the number of cells in the cell aggregate. In one or more embodiments, the concentration of the precursor of AGEs (such as the $\alpha$-dicarbonylcompound) added is 0.01 nM to 100 mM. In one or more embodiments, the concentration of the precursor of AGEs is 0.1 nM or more, 1 nM or more, 10 nM or more, 100 nM or more, or 1 mM or more. Furthermore, the concentration of the precursor of AGEs is 100 mM or less, 50 mM or less, 40 mM, 30 mM, 20 mM, 15 mM, 10 mM or less, or 5 mM or less.

In one or more embodiments, the time of contact between the cell aggregate and the precursor of AGEs (i.e., the treatment time of the cell aggregate with the precursor of AGEs) in the proliferation promoting method of the present disclosure may be appropriately determined in accordance with the number of cells in the cell aggregate. In one or more embodiments, the contact time is 1 minute or more, 5 minutes or more, 10 minutes or more, or 15 minutes or more. Furthermore, the contact time is 48 hours or less, 36 hours or less, 30 hours or less, 28 hours or less, 26 hours or less, or 24 hours or less. In the present disclosure, the "time of contact between the cell aggregate and the precursor of AGEs" indicates the time it takes from the addition of the precursor of AGEs to a culture medium to the replacement of the culture medium.

When animal-derived cells are to be cultured, any medium for animal cell culture may be used as a medium component of a culture medium. Examples of the medium include the following: Dulbecco's Modified Eagle's Medium (DMEM); Ham's Nutrient Mixture F12; DMEM/F12 Medium; McCoy's 5AMedium; Eagle's Minimum Essential Medium (EMEM); alpha Modified Eagle's Minimum Essential Medium ($\alpha$MEM); Minimum Essential Medium (MEM); RPMI 1640 Medium; Iscove's Modified Dulbecco's Medium (IMDM); MCDB 131 Medium; William's Medium E; IPL 41 Medium; Fischer's Medium; StemPro34 (manufactured by Invitrogen); X-VIVO 10 (manufactured by Cambrex Corporation); X-VIVO 15 (manufactured by Cambrex Corporation); HPGM (manufactured by Cambrex Corporation); StemSpan H3000 (manufactured by STEMCELL Technologies Inc.); StemSpanSFEM (manufactured by STEMCELL Technologies Inc.); Stemline II (manufactured by Sigma-Aldrich); QBSF-60 (manufactured by Quality Biological, Inc.); StemPro hESC SFM (manufactured by Invitrogen); Essential 8 (registered trademark) Medium (manufactured by Gibco); mTeSR1 or mTeSR2 Medium (manufactured by STEMCELL Technologies Inc.); ReproFF or ReproFF2 (manufactured by ReproCELL, Inc.); StemFit (registered trademark) AK02N (manufactured by Takara Bio Inc.); PSGro hESC/iPSC Medium (manufactured by System Biosciences, LLC); Human ES/iPS Cell Medium (xeno-free); NutriStem (registered trademark) Medium (manufactured by Biological Industries); CSTI-7 Medium (manufactured by Cell Science & Technology Institute, Inc.); MesenPRO RS Medium (manufactured by Gibco); MF-medium (registered trademark) Mesenchymal Stem Cell Growth Medium (manufactured by TOYOBO CO., LTD.); Mesenchymal Stem Cell Basal Medium, Mesenchymal Stem Cell Growth Medium 2 (manufactured by Takara Bio Inc.); Sf-900 II (manufactured by Invitrogen); and Opti-Pro (manufactured by Invitrogen).

When plant-derived cells are to be cultured, a medium component of a culture medium used in the cell culture method of the present disclosure may be, e.g., a basal medium for plant tissue culture. Examples of the basal medium include Murashige and Skoog (MS) Medium, Linsmaier and Skoog (LS) Medium, White's Medium, Gamborg's B5 Medium, Nitsch Medium, Heller Medium, and Morel Medium. Alternatively, a modified medium may be prepared by adjusting the concentration of the basal medium to optimal levels (e.g., by reducing the ammonia nitrogen concentration by half), and then plant growth-regulating substances (plant hormones) such as auxins, and optionally cytokinins, may be added at an appropriate concentration to the modified medium. The resulting medium may also be used in the cell culture method of the present disclosure.

The above media may be further supplemented with, e.g., caseinolytic enzyme, corn steep liquor, or vitamins as needed Examples of the auxins include, but are not limited to, 3-Indoleacetic acid (IAA), 3-Indolebutyric acid (IBA), 1-Naphthaleneacetic acid (NAA), and 2,4-Dichlorophenoxyacetic acid (2,4-D). The auxins may be added, e.g., at a concentration of about 0.1 to about 10 ppm to the media. Examples of the cytokinins include, but are not limited to, kinetin, benzyladenine (BA), and zeatin. The cytokinins may be added, e.g., at a concentration of about 0.1 to about 10 ppm to the media.

In one or more embodiments, the proliferation promoting method of the present disclosure includes further adding a ROCK inhibitor to a culture medium. In one or more embodiments, the ROCK inhibitor is preferably added to a culture medium that replaces the medium that was subjected to the treatment with the precursor of AGEs. In one or more embodiments, from the viewpoint of promoting the production of the ECM of cells and further improving the proliferative capacity of the cells, the proliferation promoting method of the present disclosure includes culturing the cell aggregate that has been in contact with (i.e., treated with) the precursor of AGEs preferably in a culture medium that contains the ROCK inhibitor, and more preferably in a culture medium that contains the ROCK inhibitor and is substantially free of the precursor of AGEs. In the present disclosure, the "culture medium that is substantially free of the precursor of AGEs" indicates that the concentration of the precursor of AGEs in the culture medium is 0.001 nM or less, 0.0001 nM or less, or substantially 0 nM.

In one or more embodiments, the ROCK inhibitor may be a known ROCK inhibitor. In one or more embodiments, the concentration of the ROCK inhibitor in the culture medium is 1 μM to 100 μM, preferably 1 μM to 50 μM, or approximately 10 μM.

In one or more embodiments, the proliferation promoting method of the present disclosure may include replacing the culture medium to which the precursor of AGEs has been added with a culture medium containing the ROCK inhibitor. In one or more embodiments, a replacement culture medium contains the ROCK inhibitor and a medium, and preferably contains the ROCK inhibitor and a medium but does not contain the precursor of AGEs.

In one or more embodiments, the culture medium may contain a specific compound from the viewpoint of efficiently suspending the cell aggregate formed and improving the cell proliferation efficiency. When the cell aggregate contains stem cells, the culture medium preferably contains a specific compound, as will be described later. In one or more non-limiting embodiments, the compounds disclosed in WO 2014/017513 and the following compounds may be used as a specific compound

The specific compound is not particularly limited and may be, e.g., a polymer compound, and preferably a polymer compound having an anionic functional group. Examples of the anionic functional group include a carboxy group, a sulfo group, a phosphate group, and salts thereof. The anionic functional group is preferably a carboxy group or its salt. The polymer compound may have one or more selected from the list of the anionic functional group.

Examples of the specific compound are not particularly limited and preferably include polysaccharides formed by polymerization of 10 or more monosaccharides (e.g., triose, tetrose, pentose, hexose, and heptose), and more preferably include acidic polysaccharides having an anionic functional group. In this case, any acidic polysaccharide that has an anionic functional group in the structure can be used Examples of the acidic polysaccharide include polysaccharides having a uronic acid (e.g., glucuronic acid, iduronic acid, galacturonic acid, or mannuronic acid), polysaccharides having a sulfate group or a phosphate group in a part of the structure, and polysaccharides having both structures. Moreover, examples of the acidic polysaccharide include not only naturally occurring polysaccharides, but also polysaccharides produced by microorganisms, polysaccharides produced by genetic engineering, and polysaccharides artificially synthesized using enzymes. More specifically, the acidic polysaccharides may be composed of one or more selected from the group consisting of hyaluronic acid, gellan gum, deacylated gellan gum (also referred to as DAG in the following), rhamsan gum, diutan gum, xanthan gum, carrageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and salts thereof The polysaccharides are preferably composed of hyaluronic acid, DAG, diutan gum, xanthan gum, carrageenan, or salts thereof, and more preferably composed of DAG because it can be used at a low concentration to suspend particles, and can also facilitate the recovery of the particles. Examples of the salt include salts of alkali metals such as lithium, sodium, and potassium, salts of alkaline-earth metals such as calcium, barium, and magnesium, and salts of aluminum, zinc, steel, iron, ammonium, organic base, and amino acid

The weight average molecular weight of these polymer compounds (such as polysaccharides) is preferably 10,000 to 50,000,000, more preferably 100,000 to 20,000,000, and further preferably 1,000,000 to 10,000,000. For example, the molecular weight can be measured in terms of pullulan by gel permeation chromatography (GPC). Further, phosphorylated DAG may also be used The phosphorylation can be performed by a known method

In one or more embodiments, a plurality of types (preferably two types) of the polysaccharides can be used in combination. The combination of the polysaccharides is not particularly limited and preferably includes at least DAG or its salt. The preferred combination of the polysaccharides includes DAG or its salt and polysaccharides other than DAG or its salt (e.g., xanthan gum, alginic acid, carrageenan, diutan gum, methylcellulose, locust bean gum, or salts thereof). Examples of the specific combination of the polysaccharides include, but are not limited to, DAG and rhamsan gum, DAG and diutan gum, DAG and xanthan gum, DAG and carrageenan, DAG and xanthan gum, DAG and locust bean gum, DAG and κ-carrageenan, DAG and sodium alginate, and DAG and methylcellulose.

More preferred examples of the specific compound include hyaluronic acid, deacylated gellan gum, diutan gum, carrageenan, xanthan gum, and salts thereof Among them, deacylated gellan gum or its salt is preferred In the present disclosure, the deacylated gellan gum is a linear high molecular weight polysaccharide containing four molecules of sugar: 1,3-linked glucose, 1,4-linked glucuronic acid, 1,4-linked glucose, and 1,4-linked rhamnose, as a constitutional

unit, and is represented by the following formula (I), where $R^1$ and $R^2$ are each a hydrogen atom and n is an integer of 2 or more. In the formula, $R^1$ may include a glyceryl group and $R^2$ may include an acetyl group. The content of the acetyl group and the glyceryl group is preferably 10% or less, more preferably 1% or less.

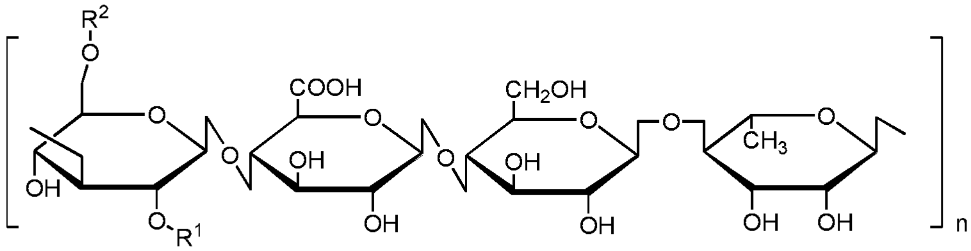

(I)

In one or more embodiments, the deacylated gellan gum may be, e.g., commercially available products such as "KELCOGEL (registered trademark) CG-LA" manufactured by Sansho Co., Ltd. and "KELCOGEL (registered trademark)" manufactured by San-Ei Gen F.F.I., Inc. In one or more embodiments, native gellan gum may be, e.g., "KELCOGEL (registered trademark) HT" manufactured by San-Ei Gen F.F.I., Inc.

In one or more embodiments, the concentration of the specific compound in the culture medium (mass/volume%, hereinafter simply referred to as %) is 0.0005% to 1.0%, preferably 0.001% to 0.4%, more preferably 0.005% to 0.1%, and further preferably 0.005% to 0.05%. When the specific compound is deacylated gellan gum, in one or more embodiments, the concentration of the deacylated gellan gum is 0.001% to 1.0%, preferably 0.003% to 0.5%, more preferably 0.005% to 0.1%, even more preferably 0.01% to 0.05%, and further preferably 0.02% to 0.05%. When the specific compound is xanthan gum, in one or more embodiments, the concentration of the xanthan gum is 0.001% to 5.0%, preferably 0.01% to 1.0%, more preferably 0.05% to 0.6%, and further preferably 0.3% to 0.6%. When the specific compound is a mixture of κ-carrageenan and locust bean gum, in one or more embodiments, the concentration of the mixture is 0.001% to 5.0%, preferably 0.005% to 1.0%, more preferably 0.01% to 0.1%, and further preferably 0.03% to 0.05%. When the specific compound is native gellan gum, in one or more embodiments, the concentration of the native gellan gum is 0.05% to 1.0%, and preferably 0.05% to 0.1%.

When a plurality of types (preferably two types) of the polysaccharides are used in combination, the concentration of the polysaccharides can be appropriately determined so that the cell aggregate can remain suspended in the culture medium in the static state. For example, in the combination of DAG or its salt and polysaccharides other than DAG or its salt, the concentration of DAG or its salt is, e.g., 0.005 to 0.02%, and preferably 0.01 to 0.02%. The concentration of polysaccharides other than DAG or its salt is, e.g., 0.005 to 0.4%, and preferably 0.1 to 0.4%. Examples of the combination of specific concentration ranges are as follows.

DAG or its salt: 0.005 to 0.02% (preferably 0.01 to 0.02%)

Polysaccharides other than DAG

xanthan gum: 0.1 to 0.4%

sodium alginate: 0.1 to 0.4%

locust bean gum: 0.1 to 0.4%

methylcellulose: 0.1 to 0.4% (preferably 0.2 to 0.4%)

carrageenan: 0.05 to 0.1%

diutan gum: 0.05 to 0.1%

The concentration can be calculated by the following formula:

$$\text{Concentration (\%)} = \text{mass of specific compound (g)} / \text{volume of liquid (ml)} \times 100$$

The specific compound can be further converted to another derivative by a chemical synthesis method, and the derivative thus obtained can also be effectively used in the preparation method of the present disclosure. Specifically, the present disclosure can use the derivatives of deacylated gellan gum that are obtained by substituting the hydroxyl group of R and/or $R^2$ of the compound represented by the formula (I) with, e.g., a $C_{1-3}$ alkoxy group, a $C_{1-3}$ alkylsulfonyl group, a monosaccharide residue such as glucose or fructose, an oligosaccharide residue such as sucrose or lactose, or an amino acid residue such as glycine or arginine. The compound can also be crosslinked by using a crosslinker such as 1-ethyl-3-(3-dimethylaminopropyl)carbodiimide (EDC).

[Method for preparing cell aggregate]

Another aspect of the present disclosure relates to a method for preparing a cell aggregate. The method includes adding a precursor of AGEs to a culture medium containing a cell aggregate, and culturing the cell aggregate in the culture medium to which the precursor of AGEs has been added

In the preparation method of the present disclosure, cells, cell aggregates, the precursor of AGEs, the ROCK inhibitor, the concentrations of these substances added, and the culture conditions are the same as the proliferation promoting method of the present disclosure.

[Suspension culture method]

Another aspect of the present disclosure relates to a method for culturing stem cells in suspension. The method includes culturing stem cells in suspension to form a cell aggregate, and culturing the cell aggregate in suspension in a culture medium containing a precursor of AGEs.

In the suspension culture method of the present disclosure, stem cells, cell aggregates, the precursor of AGEs, the ROCK inhibitor, the concentrations of these substances added, and the culture conditions are the same as the proliferation promoting method of the present disclosure.

[Method for unraveling ECM shell]

As described above, the present disclosure allows the ECM shell (fibrillar ECM / fibrotic ECM) formed on the surface of a cell aggregate to be non-fibrillated (unraveled). Thus, another aspect of the present disclosure relates to a method for unraveling an ECM shell of a cell aggregate (a method for bringing a fibrotic ECM an ECM shell covering a surface of a cell aggregate back to a near-normal ECM or non-fibrotic ECM). The method includes culturing cells to form a cell aggregate, and adding a precursor of AGEs to a culture medium containing the cell aggregate.

In the ECM shell unraveling method of the present disclosure, cells, cell aggregates, the precursor of AGEs, the ROCK inhibitor, the concentrations of these substances added, and the culture conditions are the same as the proliferation promoting method of the present disclosure.

As described above, when the proliferation of cells forming cell aggregates is stopped or the proliferation rate is reduced (causing a decrease in proliferative capacity), the decreased proliferative capacity of the cells in the cell aggregates can be restored by bringing the α-dicarbonyl compound such as methylglyoxal, glyoxal, or 3-Deoxyglucosone into contact with the cell aggregates. Thus, another aspect of the present disclosure relates to a method for promoting cell proliferation and a method for preparing a cell aggregate. The methods include adding an α-dicarbonyl compound to a culture medium containing a cell aggregate, and culturing the cell aggregate in the culture medium to which the α-dicarbonylcompound has been added. Moreover, another aspect of the present disclosure relates to a suspension culture method. The method includes culturing stem cells in suspension to form a cell aggregate, and culturing the cell aggregate in suspension in a culture medium containing an α-dicarbonylcompound. This aspect can be performed in the same manner as the aspect using the precursor of AGEs.

In this aspect, the α-dicarbonylcompound may be or may not be the precursor of AGEs. In one or more embodiments, the α-dicarbonylcompound includes methylglyoxal, glyoxal, 3-Deoxyglucosone, malondialdehyde, butanedione, 1,2-Cyclohexanedione, phenylglyoxal, 4-Fluorophenylglyoxal, 4-Nitrophenylglyoxal, and 4-Hydroxyphenylglyoxal.

[Cell aggregate of stem cells]

Another aspect of the present disclosure relates to a cell aggregate of stem cells obtained by the preparation method and/or ECM shell unraveling method of the present disclosure.

According to the preparation method of the present disclosure, in one or more embodiments, stem cells can proliferate while maintaining their undifferentiated state in a cell. aggregate. Thus, in one or more embodiments, the cell aggregate of the present disclosure is composed of substantially undifferentiated stem cells.

[Anti-fibrillating agent for cell aggregate]

Another aspect of the present disclosure relates to an anti-fibrillating agent (anti-fibrotic agent) for a cell aggregate (i.e., an agent for bringing a fibrotic ECM covering a cell aggregate back to a near-normal ECM or non-fibrotic ECM). The anti-fibrillating agent contains a precursor of AGEs.

The anti-fibrillating agent of the present disclosure can be in any form of solid, powder, or liquid The anti-fibrillating agent may further contain other components unless they interfere with the effects of the present disclosure.

Another aspect of the present disclosure relates to an anti-fibrillating agent for a cell aggregate. The anti-fibrillating agent contains an α-dicarbonyl compound. In this aspect, the α-dicarbonyl compound may be or may not be the precursor of AGEs. In one or more embodiments, the α-dicarbonyl compound includes methylglyoxal, glyoxal, 3-Deoxyglucosone, malondialdehyde, butanedione, 1,2-Cyclohexanedione, phenylglyoxal, 4-Fluorophenylglyoxal, 4-Nitrophenylglyoxal, and 4-Hydroxyphenylglyoxal. The anti-fibrillating agent of this aspect can be in any form of solid, powder, or liquid The anti-fibrillating agent may further contain other components unless they interfere with the effects of the present disclosure.

[Kit for culturing cell aggregate]

Another aspect of the present disclosure relates to a kit for culturing a cell aggregate. The kit contains a precursor of AGEs and a ROCK inhibitor.

The precursor of AGEs and the ROCK inhibitor can be in any form of solid, powder, or liquid In one or more embodiments, the precursor of AGEs and the ROCK inhibitor in the kit of the present disclosure are placed in separate containers.

Another aspect of the present disclosure relates to a kit for culturing a cell aggregate. The kit contains an α-dicarbonyl compound and a ROCK inhibitor. In this aspect, the α-dicarbonyl compound may be or may not be the precursor of AGEs. In one or more embodiments, the α-dicarbonylcompound includes methylglyoxal, glyoxal, 3-Deoxyglucosone, malondialdehyde, butanedione, 1,2-Cyclohexanedione, phenylglyoxal, 4-Fluorophenylglyoxal, 4-Nitrophenylglyoxal, and 4-Hydroxyphenylglyoxal. In this aspect, the α-dicarbonyl compound and the ROCK inhibitor can be in any form of solid, powder, or liquid

In one or more embodiments, the kit of the present disclosure may further contain a medium component for culturing a cell aggregate and/or the specific compound The medium component and the specific compound are as described above.

Hereinafter, a non-limiting embodiment of the method of the present disclosure will be described

[Embodiment 1]

In Embodiment 1, stem cells are iPS cells, and methylglyoxal is used as a precursor of AGEs.

First, iPS cells are seeded in a medium and cultured in suspension, so that cell aggregates of the iPS cells are formed

In one or more embodiments, the concentration of the cells to be seeded is $1 \times 10$ cells/cm$^2$ to $1 \times 10^8$ cells/cm$^2$, preferably $1 \times 10^2$ cells/cm$^2$ to $1 \times 10^6$ cells/cm$^2$, and more preferably $1 \times 10^3$ cells/cm$^2$ to $1 \times 10^5$ cells/cm$^2$ In one or more embodiments, the culture temperature is 30 to 40°C, and preferably about 37°C. In one or more embodiments, the $CO_2$ concentration during culture is 1 to 10%, and preferably about 5%.

Any of the above culture media can be used as a culture medium for suspension culture.

In one or more embodiments, the culture medium in which the cells are seeded preferably contains a ROCK inhibitor to reduce cell death after seeding. In one or more embodiments, the concentration of the ROCK inhibitor in the culture medium is 1 μM to 100 μM, preferably 1 μM to 50 μM, or approximately 10 μM.

In one or more embodiments, the iPS cells are cultured in the culture medium containing the ROCK inhibitor for 12 hours or more, 15 hours or more, 18 hours or more, 21 hours or more, or 24 hours or more. Although the ROCK inhibitor can always be present in the culture medium, the upper limit of the culture time in the presence of the ROCK inhibitor is, e.g., 72 hours or less, 48 hours or less, 36 hours or less, 33 hours or less, 30 hours or less, 27 hours or less, or 24 hours or less.

After culturing the iPS cells in the culture medium containing the ROCK inhibitor, the culture medium may be replaced as needed. In one or more embodiments, a replacement culture medium may be any of the above culture media. In one or more embodiments, the replacement culture medium may contain or may not contain the ROCK inhibitor. In one or more embodiments, whether or not the ROCK inhibitor will be added to the culture medium may depend on the ECM secretion capacity of the cells to be cultured

Next, methylglyoxal is added to the culture medium in which the cell aggregates are formed, and suspension culture is further performed. The contact between methylglyoxal and the cell aggregates can promote the re-proliferation of the iPS cells in the cell aggregates and improve the proliferative capacity of the iPS cells. Consequently, the cell aggregates can have a larger diameter and more stem cells.

The amount of methylglyoxal added may be appropriately determined by the concentration of the seeded cells and the culture conditions. In one or more embodiments, the concentration of methylglyoxal in the culture medium is 0.1 nM or more, 1 nM or more, 10 nM or more, 100 nM or more, or 1 mM or more. Furthermore, the concentration of methylglyoxal is 100 mM or less, 50 mM or less, 10 mM or less, or 5 mM or less.

In one or more embodiments, the culture time of the cell aggregates in the culture medium containing methylglyoxal (i.e., the treatment time of the cell aggregates with methylglyoxal) may be appropriately determined in accordance with, e.g., the number of cells in the cells aggregates. In one or more embodiments, the contact time is 1 minute or more, 5 minutes or more, 10 minutes or more, or 15 minutes or more. Furthermore, the contact time is 48 hours or less, 36 hours or less, 30 hours or less, 28 hours or less, 26 hours or less, or 24 hours or less.

In terms of production cost and production efficiency, methylglyoxal may be added either after the iPS cells are cultured for a predetermined period of time or after the proliferation of the cell aggregates can hardly be confirmed or is substantially stopped.

In one or more embodiments, the culture medium to which methylglyoxal is added may contain or may not contain the ROCK inhibitor.

After adding methylglyoxal to the culture medium, the culture medium may be replaced as needed. In one or more embodiments, a replacement culture medium may be any of the above culture media. In one or more embodiments, the replacement culture medium may contain or may not contain reactive aldehyde such as methylglyoxal In terms of manufacturing cost, it is preferable that the replacement culture medium does not contain reactive aldehyde. In one or more embodiments, the replacement culture medium preferably contains the ROCK inhibitor from the viewpoint of promoting the production of the extracellular matrix of cells in the cell aggregates. In one or more embodiments, the concentration of the ROCK inhibitor in the culture medium is 1 $\mu$M to 100 $\mu$M, preferably 1 $\mu$M to 50 $\mu$M, or approximately 10 $\mu$M.

Methylglyoxal may be added once or repeatedly. Repeated addition of methylglyoxal can result in cell aggregates with a larger cell diameter.

The cell aggregates of the iPS cells obtained by the above suspension culture remain undifferentiated. These cell aggregates of the iPS cells may be dissociated into small aggregates, scaled up, and mass cultured in the undifferentiated state using, e.g., a bioreactor. Alternatively, the cell aggregates of the iPS cells may be induced to differentiate into cells of target organs.

In Embodiment 1, the precursor of AGEs is methylglyoxal. Embodiment 1 can be performed in the same manner as described above if the precursor of AGEs is not methylglyoxal but is selected from, e.g., the other $\alpha$-dicarbonyl compounds and $\alpha$-hydroxy aldehyde compounds.

[Embodiment 2]

In Embodiment 2, stem cells are mesenchymal stem cells, and methylglyoxal is used as a precursor of AGEs.

First, mesenchymal stem cells are seeded in a medium and cultured in suspension, so that cell aggregates of the mesenchymal stem cells are formed.

In one or more embodiments, the concentration of the cells to be seeded is $1 \times 10$ cells/cm$^2$ to $1 \times 10^8$ cells/cm$^2$, preferably $1 \times 10^2$ cells/cm$^2$ to $1 \times 10^6$ cells/cm$^2$, and more preferably $1 \times 10^3$ cells/cm$^2$ to $1 \times 10^5$ cells/cm$^2$. In one or more embodiments, the culture temperature is 30 to 40°C, and preferably about 37°C. In one or more embodiments, the $CO_2$ concentration during culture is 1 to 10%, and preferably about 5%.

Any of the above culture media can be used as a culture medium in which the cells are seeded. In one or more embodiments, the culture medium in which the cells are seeded preferably does not contain a ROCK inhibitor in terms of manufacturing cost.

The culture medium may be replaced as needed In one or more embodiments, a replacement culture medium may contain or may not contain the ROCK inhibitor. In terms of manufacturing cost, it is preferable that the replacement culture medium does not contain the ROCK inhibitor.

Next, methylglyoxal is added to the medium in which the cell aggregates are formed, and suspension culture is further performed The mesenchymal stem cells hardly proliferate when they form cell aggregates. By bringing methylglyoxal into contact with the cell aggregates, the mesenchymal stem cells are able to proliferate in suspension culture.

The amount of methylglyoxal added may be appropriately determined by the concentration of the seeded cells and the culture conditions. In one or more embodiments, the concentration of methylglyoxal in the culture medium is 0.1 nM or more, 0.5 nM or more, 1 nM or more, 10 nM or more, 0.1 mM or more, or 1 mM or more. Furthermore, the concentration of methylglyoxal is 30 mM or less, 20 mM or less, 10 mM or less, 5 mM or less, 1 mM or less, or 0.1 mM or less.

In one or more embodiments, the culture time of the cell aggregates in the culture medium containing methylglyoxal (i.e., the treatment time of the cell aggregates with methylglyoxal) may be appropriately determined in accordance with, e.g., the number of cells in the cells aggregates. In one or more embodiments, the contact time is 1 minute or more, 5 minutes or more, 10 minutes or more, or 15 minutes or more. Furthermore, the contact time is 48 hours or less, 36 hours or less, 30 hours or less, 28 hours or less, 26 hours or less, or 24 hours or less.

In terms of improving the proliferation rate of the mesenchymal stem cells, the ROCK inhibitor is preferably added to the medium at the same time as and/or after the addition of methylglyoxal In one or more embodiments, the concentration of the ROCK inhibitor in the culture medium is 1 $\mu$M to 100 $\mu$M, preferably 1 $\mu$M to 50 $\mu$M, or approximately 10 $\mu$M.

After adding methylglyoxal to the culture medium, the culture medium may be replaced as needed. In one or more embodiments, a replacement culture medium may contain or may not contain reactive aldehyde such as methylglyoxal. In terms of manufacturing cost, it is preferable that the replacement culture medium does not contain reactive aldehyde. In one or more embodiments, the replacement culture medium may contain or may not contain the ROCK inhibitor. In terms of improving the proliferation rate of the mesenchymal stem cells, it is preferable that the replacement culture medium contains the ROCK inhibitor. In one or more embodiments, the concentration of the ROCK inhibitor in the culture medium is 1 μM to 100 μM, preferably 1 μM to 50 μM, or approximately 10 μM.

Methylglyoxal may be added once or repeatedly. Repeated addition of methylglyoxal can result in cell aggregates with a larger cell diameter.

The cell aggregates of the mesenchymal stem cells obtained by the above suspension culture remain undifferentiated. These cell aggregates of the mesenchymal stem cells may be dissociated into small aggregates, scaled up, and mass cultured in the undifferentiated state using, e.g., a bioreactor. Alternatively, the cell aggregates of the mesenchymal stem cells may be induced to differentiate into cells of target organs.

In Embodiment 2, the precursor of AGEs is methylglyoxal. Embodiment 2 can be

performed in the same manner as described above if the precursor of AGEs is not methylglyoxal but is selected from, e.g., the other α-dicarbonyl compounds and α-hydroxy aldehyde compounds.

The present disclosure further relates to one or more non-limiting embodiments in the following.

[A1] A method for promoting proliferation of cells,

the method comprising:
adding a precursor of advanced glycation end products (AGEs) to a culture medium containing a cell aggregate; and
culturing the cell aggregate in the culture medium to which the precursor of AGEs has been added.

[A2] The method according to [A1], comprising:
adding a ROCK inhibitor to the culture medium at the same time as or after the addition of the precursor of AGEs.
[A3] A method for preparing a cell aggregate,

the method comprising:
adding a precursor of AGEs to a culture medium containing a cell aggregate; and
culturing the cell aggregate in the culture medium to which the precursor of AGEs has been added.

[A4] The method according to [A3], comprising:
adding a ROCK inhibitor to the culture medium at the same time as or after the addition of the precursor of AGEs.
[A5] The method according to any one of [A1] to [A4], wherein the cells are stem cells.
[A6] The method according to any one of [A1] to [A5], wherein the precursor of AGEs is at least one of an α-dicarbonyl compound or an α-hydroxy aldehyde compound.
[A7] The method according to [A6], wherein the α-dicarbonyl compound is selected from the group consisting of methylglyoxal, glyoxal, 3-Deoxyglucosone, malondialdehyde, butanedione, 1,2-Cyclohexanedione, phenylglyoxal, 4-Fluorophenylglyoxal, 4-Nitrophenylglyoxal, 4-Hydroxyphenylglyoxal, and combinations thereof.
[A8] The method according to [A6] or [A7], wherein the α-hydroxy aldehyde compound is selected from the group consisting of glyceraldehyde, glycolaldehyde, lactaldehyde, 3-Hydroxybutyraldehyde, and combinations thereof
[A9] The method according to any one of [A1] to [A8], comprising:
preparing the culture medium containing the cell aggregate.
[A10] The method according to [A9], wherein the preparation of the culture medium containing the cell aggregate includes seeding monodispersed cells in a culture medium, and culturing the seeded cells in suspension to form a cell aggregate.
[A11] The method according to [A10], wherein the cells to be seeded are iPS cells.
[A12] The method according to [A10] or [A11], wherein the culture medium in which the cells are seeded contains a ROCK inhibitor.
[A13] The method according to [A10], wherein the cells to be seeded are somatic stem cells.
[A14] The method according to [A13], wherein the culture medium in which the cells are seeded does not contain a ROCK inhibitor.
[A15] The method according to any one of [A9] to [A14], wherein the preparation of the culture medium containing the cell aggregate includes replacing the culture medium after seeding the cells.
[A16] The method according to [A15], wherein a culture medium for the replacement does not contain a ROCK inhibitor.

[A17] The method according to any one of [A1] to [A16], wherein a concentration of the precursor of AGEs added is 0.01 nM to 100 mM
[A18] The method according to any one of [A1] to [A17], comprising:
replacing the culture medium to which the precursor of AGEs has been added with a culture medium that does not contain the precursor of AGEs.
[A19] The method according to [A18], wherein the culture medium is replaced 1 minute to 48 hours after the addition of the precursor of AGEs.
[A20] The method according to [A18] or [A19], wherein a culture medium for the replacement contains a ROCK inhibitor.
[A21] The method according to [A20], wherein a concentration of the ROCK inhibitor in the culture medium is 1 μM to 100 μM.
[B1] A method for unraveling an ECM shell covering a surface of a cell aggregate,

the method comprising:
culturing cells to form a cell aggregate; and
adding a precursor of AGEs to a culture medium containing the cell aggregate.

[B2] The method according to [B1], wherein the cells are stem cells.
[B3] The method according to [B1] or [B2], wherein the precursor of AGEs is at least one of an α-dicarbonyl compound or an α-hydroxy aldehyde compound
[B4] The method according to [B3], wherein the α-dicarbonyl compound is selected from the group consisting of methylglyoxal, glyoxal, 3-Deoxyglucosone, malondialdehyde, butanedione, 1,2-Cyclohexanedione, phenylglyoxal, 4-Fluorophenylglyoxal, 4-Nitrophenylglyoxal, 4-Hydroxyphenylglyoxal, and combinations thereof.
[B5] The method according to [B3] or [B4], wherein the α-hydroxy aldehyde compound is selected from the group consisting of glyceraldehyde, glycolaldehyde, lactaldehyde, 3-Hydroxybutyraldehyde, and combinations thereof
[B6] The method according to any one of [B 1] to [B5], wherein the formation of the cell aggregate includes seeding monodispersed cells in a culture medium, and culturing the seeded cells in suspension to form a cell aggregate.
[B7] The method according to [B6], wherein the cells to be seeded are iPS cells.
[B8] The method according to [B6] or [B7], wherein the culture medium in which the cells are seeded contains a ROCK inhibitor.
[B9] The method according to [B6], wherein the cells to be seeded are somatic stem cells.
[B10] The method according to [B6] or [B9], wherein the culture medium in which the cells are seeded does not contain a ROCK inhibitor.
[B11] The method according to any one of [B 1] to [B10], wherein the formation of the cell aggregate includes replacing the culture medium after seeding the cells.
[B12] The method according to [B11], wherein a culture medium for the replacement does not contain a ROCK inhibitor.
[B13] The method according to any one of [B 1] to [B12], wherein a concentration of the precursor of AGEs added is 0.01 nM to 100 mM
[B14] The method according to any one of [B1] to [B13], comprising:
replacing the culture medium to which the precursor of AGEs has been added with a culture medium that does not contain the precursor of AGEs.
[B15] The method according to [B14], wherein the culture medium is replaced 1 minute to 48 hours after the addition of the precursor of AGEs.
[B16] The method according to [B14] or [B15], wherein a culture medium for the replacement contains a ROCK inhibitor.
[B17] The method according to [B16], wherein a concentration of the ROCK inhibitor in the culture medium is 1 μM to 100 μM.
[C1] A cell aggregate obtained by the method according to any one of [A3] to [A21] and [B1] to [B17],
[D1] An anti-fibrillating agent for a cell aggregate (an agent for bringing a fibrotic ECM covering a cell aggregate back to a near-normal ECM or non-fibrotic ECM),
the agent comprising a precursor of AGEs.
[D2] The agent according to [D1], wherein the agent is solid, powder, or liquid [E 1] A kit for culturing a cell aggregate,

the kit comprising:
a precursor of AGEs and a ROCK inhibitor.

[E2] The kit according to [E 1], further comprising a culture medium component.

Hereinafter, the present disclosure will be described in more detail by way of test examples. The test examples are illustrative and not intended to limit the present disclosure.

Examples

[Test Example 1: Aggregate culture 1 of iPS cells]

iPSC aggregates were cultured under the following culture conditions. FIGS. 1A and 1B show the results.

[Culture conditions]

Cell: iPSCs (D2 strain, Np43)
Medium: StemFit (registered trademark) AK02N (manufactured by Takara Bio Inc.)
Culture vessel: 96 well V-bottom plate (MS-9096V manufactured by Sumitomo Bakelite Co., Ltd.)
Cell density: 1316 cells/cm$^2$ (5 $\times$ 10$^2$ cells/wells)
Culture condition: 37°C, 5% $CO_2$ incubator
Amount of medium: 150 μl/well
Culture period: 7 days (Methylglyoxal was added once after the medium was replaced on the 4th day of culture (Day 4).)
Medium on Day 0: the above medium containing 10 μM ROCK inhibitor Replacement of medium: 150 μl/well
Medium for replacement: the above medium containing no ROCK inhibitor Added reagent: Methylglyoxal Solution (product code: M0252, 1.17 g/mL, 16.25 M, manufactured by Sigma-Aldrich)
Methylglyoxal (MG) concentration: 0, 0.1, 1, 10, 100 mM
Imaging: photomicrography on the 4th day of culture (Day 4) (before addition of MG) and the 5th, 6th, and 7th day of culture (Day 5, 6, 7) (after addition of MG)

FIGS. 1A and 1B show an example of photomicrographs of iPS cell aggregates before the addition of MG (Day 4) and after the addition of MG (Day 5, 6, 7). FIG. 1B is a partially enlarged view of FIG. 1A

The proliferation of cells in the iPS cell aggregate (i.e., an increase in the diameter of the cell aggregate) could hardly be observed on Day 4 (before the addition of MG). This iPS cell aggregate was treated with MG. Consequently, the periphery (outer circumferential portion) of the cell aggregate became non-fibrillated (unraveled), and thus the cell binding properties could be reduced, as compared to the iPS cell aggregate that was not treated with MG (control). Moreover, the cell binding properties in the periphery of the cell aggregate tended to be further reduced with an increase in the MG concentration.

It is considered that as a cell aggregate grows to a certain size, cell migration becomes lower, which in turn may reduce the proliferation of cells in the center of the cell aggregate. As described above, since the iPS cell aggregate is cultured in the presence of MG, the cell binding properties in the periphery of the cell aggregate can be reduced, providing space for cells in the cell aggregate to proliferate. This can prevent a reduction in cell migration or can improve the reduced cell migration. Thus, the results indicate that the proliferative capacity of the cell aggregate can be improved

[Test Example 2: Aggregate culture 2 of iPSCs]

iPSC aggregates were cultured under the following culture conditions and procedures shown in FIG. 2. FIG. 4 shows the results.

[Culture conditions]

Cell: iPSCs (D2 strain, Np 17, feeder less)
Medium: StemFit (registered trademark) AK02N (manufactured by Takara Bio Inc.)
Culture vessel: 96 well V-bottom plate (MS-9096V manufactured by Sumitomo Bakelite Co., Ltd)
Cell density: 200 cells/well
Culture condition: 37°C, 5% $CO_2$
Amount of medium: 150 μl/well
Culture period: 10 days and (subculture) 5 days (15 days in total)

Medium on Day 0: the above medium containing 10 pM ROCK inhibitor Replacement of medium: 75 μl/well (daily)
Medium for replacement:

Day 4 to 10: the above medium containing 10 μM ROCK inhibitor
Day 1 to 3: the above medium containing no ROCK inhibitor

Methylglyoxal concentration: 0, 1.25, 2.5, 5, 10, 15, 20 mM
Added reagent: Methylglyoxal Solution (product code: M0252, 1.17 g/mL, 16.25 M, manufactured by Sigma-Aldrich)
Addition of MG: MG was added 20 minutes before the medium was replaced on the 4th day of culture (Day 4).
MG treatment time: 20 minutes
Imaging: video observation with BioStudio (manufactured by NIKON ENGINEERING CO., LTD.) after addition ofMG

[Test Example 3: Aggregate culture 3 of iPSCs]

Test Example 3 was performed in the same manner as Test Example 2 (aggregate culture 2 of iPSCs) except that the culture conditions were changed as follows and the procedures shown in FIG. 3 were used FIG. 4 shows the results.

[Culture conditions]

Addition of MG: MG was added after the medium was replaced on the 4th day of culture (Day 4).
MG treatment time: 24 hours
Medium for replacement:

Day 4 to 10: the above medium containing 10 μM ROCK inhibitor
Day 1 to 3: the above medium containing no ROCK inhibitor

FIG. 4 shows an example of photomicrographs of iPS cell aggregates before the addition of MG (Day 4) and after the addition of MG (Day 10). The proliferation of cells in the iPS cell aggregate (i.e., an increase in the diameter of the cell aggregate) could hardly be observed on Day 4 (before the addition of MG). This iPS cell aggregate was treated with MG and then cultured in the culture medium containing the ROCK inhibitor. Consequently, the periphery of the cell aggregate was found to expand slightly due to the aggregate culture in the culture medium, regardless of whether the MG treatment time was 20 minutes or 24 hours. In other words, the above treatment and culture had the effect of promoting cell proliferation.

Thus, the results indicate that the above treatment can make the ECM on the surface of the iPS cell aggregate (ECM shell) non-fibrillated, and can also promote the production of the ECM by cells, so that the proliferative capacity of the iPS cells can be restored

[Test Example 4: Aggregate culture 1 of mesenchymal stem cells (MSCs)]

MSC aggregates were cultured under the following culture conditions. The number of cells was counted on Day 4 (before the addition of MG) and on Day 9. Using the resulting number of cells, the cell proliferation rate was obtained from the following formula. FIG. 5 shows the results.

[Culture conditions]

Cell: MSCs (Np2)
Medium: Mesenchymal Stem Cell Growth Medium 2 (manufactured by Takara Bio Inc.)
Culture vessel: Micro-Space Cell Culture Plate (manufactured by Kuraray Co., Ltd), 24 well plate (manufactured by Corning)
Culture condition: 37°C, 5% $CO_2$ incubator
Amount of medium: 3 mL/well
Seeding density: $1.0 \times 10^5$ cell/well
Culture period: 9 days

Medium on Day 0: the above medium containing no ROCK inhibitor
Replacement of medium: 1.5 mL/well × 3, once every two days (Day 5, 7, 9)
Medium for replacement:

Day 2,4: the above medium containing no ROCK inhibitor
Day 5,7,9: the above medium containing 10 μM ROCK inhibitor

Added reagent: Methylglyoxal Solution (product code: M0252, 1.17 g/mL, 16.25 M, manufactured by Sigma-Aldrich)
Methylglyoxal concentration: 0, 0.0075, 0.01, 0.0125, 0.015, 0.02 mM
Addition of MG: MG was added after the medium was replaced on the 4th day of culture (Day 4).
MG treatment time: 24 hours
[Cell proliferation rate]

$$\text{Cell proliferation rate} = (\text{the number of cells on Day 9}) / (\text{the number of cells on Day 3})$$

FIG. 5 is an example of a graph of the cell proliferation rate. As shown in the graph of FIG. 5, the cell proliferation rate was 1.2 times when MG was not added (0 mM), whereas the cell proliferation rate nearly doubled by adding MG to the medium. It is considered that MSCs do not proliferate when they form cell aggregates, and subculture is required to increase the number of MSCs. However, the addition of MG to the medium allows the MSCs to proliferate even after they form cell aggregates.
The same culture was performed without the addition of the ROCK inhibitor. As a result, the cell proliferation rate was higher in the culture using the medium containing the ROCK inhibitor (i.e., the combination of MG and the ROCK inhibitor) than in the culture using the medium containing no ROCK inhibitor.
Next, MSCs were cultured under the culture conditions of Test Example 4 except that the MSCs were subjected to monolayer culture instead of aggregate culture. As a result, the cell proliferation rate did not change significantly regardless of the presence or absence of the addition ofMG and the ROCK inhibitor.

[Test Example 5: Aggregate culture 2 of MSCs]

MSC aggregates were cultured under the following culture conditions. FIG. 6 shows the results.

[Culture conditions]

Cell: MSCs (Np2)
Medium: Mesenchymal Stem Cell Growth Medium 2 (manufactured by Takara Bio Inc.)
Culture vessel: Micro-Space Cell Culture Plate (manufactured by Kuraray Co., Ltd), 24 well plate (manufactured by Corning)
Culture condition: 37°C, 5% $CO_2$ incubator
Amount of medium: 3 mL/well
Seeding density: 1.0 × $10^5$ cell/well
Culture period: 15 days
Medium on Day 0: the above medium containing no ROCK inhibitor
Replacement of medium: 1.5 mL/well × 3, once every two days
Medium for replacement:

Day 2: the above medium containing no ROCK inhibitor
Day 4,6,8,10,12,14:the above medium containing 10 μM ROCK inhibitor

Added reagent: Methylglyoxal Solution (product code: M0252, 1.17 g/mL, 16.25 M, manufactured by Sigma-Aldrich)
Methylglyoxalconcentration: 0, 1.25, 2.5, 5,10,15, 20 mM
Addition of MG: MG was added 20 minutes before the medium was replaced on the 4th day of culture (Day 4).
MG treatment time: 20 minutes
Imaging: Day 4 (before addition ofMG) and Day 15

[Test Example 6: Aggregate culture 3 of MSCs]

Test Example 6 was performed in the same manner as Test Example 5 (aggregate culture 2 of MSCs) except that the culture conditions were changed as follows. FIG. 6 shows the results.

[Culture conditions]

Addition of MG: MG was added after the medium was replaced on the 4th day of culture (Day 4).
MG treatment time: 24 hours
Medium for replacement:

Day 2,4: the above medium containing no ROCK inhibitor
Day 5, 7,9,11,13, 15: the above medium containing 10 μM ROCK inhibitor

FIG. 6 shows an example of photomicrographs of MSC cell aggregates before and after the addition of MG (Day 4 and Day 15). The proliferation of cells in the MSC cell aggregate (i.e., an increase in the diameter of the cell aggregate) could hardly be observed on Day 4 (before the addition of MG). This MSC cell aggregate was treated with MG and then cultured in the culture medium containing the ROCK inhibitor. Consequently, the periphery of the cell aggregate was found to expand slightly due to the aggregate culture in the culture medium, regardless of the MG treatment time. In other words, the above treatment and culture had the effect of promoting cell proliferation.

Thus, the results indicate that the above treatment can make the ECM on the surface of the MSC cell aggregate (ECM shell) non-fibrillated, and can also promote the production of the ECM by cells, so that the proliferative capacity of the MSCs can be restored

[Test Example 7: Aggregate culture 4 of MSCs]

Test Example 7 was performed in the same manner as Test Example 6 (aggregate culture 3 of MSCs) except that the culture conditions were changed as follows. FIG. 7 shows the results.

[Culture conditions]

Culture period: 9 days
Medium on Day 0: the above medium containing no ROCK inhibitor
Added reagent: 3-Deoxyglucosone (3-DG) (manufactured by Sigma-Aldrich)
Glyoxal Solution (GO) (manufactured by Sigma-Aldrich)
Addition of 3-DG or GO: 3-DG or GO was added after the medium was replaced on the 4th day of culture (Day 4).
3-DG or GO treatment time: 24 hours
Medium for replacement:

Day 2,4: the above medium containing no ROCK inhibitor
Day 5, 7,9: the above medium containing 10 μM ROCK inhibitor

FIG. 7 shows an example of photomicrographs of MSC cell aggregates before (Day 4) and after (Day 9) the addition of 3-DG/GO. The proliferation of cells in the MSC cell aggregate (i.e., an increase in the diameter of the cell aggregate) could hardly be observed on Day 4 (before the addition of 3-DG/GO). This MSC cell aggregate was treated with 3-DG or GO and then cultured in the culture medium containing the ROCK inhibitor. Consequently, the periphery of the cell aggregate was found to expand slightly. In other words, the above treatment and culture had the effect of promoting cell proliferation.

Thus, the results indicate that the above treatment can make the ECM on the surface of the MSC cell aggregate (ECM shell) non-fibrillated, and can also promote the production of the ECM by cells, so that the proliferative capacity of the MSCs can be restored

Similarly to Test Example 4, the number of cells was counted on Day 4 (before the treatment) and on Day 9, and the cell proliferation rate was confirmed As a result, the cell proliferation rate of the MSC cell aggregate was increased by culturing the MSC cell aggregate that had been treated with 3-DG or GO, like the MG treatment. Moreover, the cell

proliferation rate tended to be higher with an increase in the 3-DG/GO concentration.

[Test Example 8: Aggregate culture of HepG2]

HepG2 aggregates were cultured under the following culture conditions. The number of cells was counted on Day 3 (before the addition of MG) and on Day 4, and the cell proliferation rate was confirmed FIG. 8 shows the results.

[Culture conditions]

Cell: HepG2 (hepatocyte)
Medium: HepG2 Hepatocellular Carcinoma Expansion Media, Human (Cellular Engineering Technologies)
Culture vessel: 24 well plate (manufactured by Corning)
Culture condition: 37°C, 5% $CO_2$ incubator
Amount of medium: 0.5 mL/well
Culture period: 6 days
Medium on Day 0: the above medium containing no ROCK inhibitor
Medium for replacement:

Day 2, 3: the above medium containing no ROCK inhibitor
Day 4,6: the above medium containing 10 $\mu$M ROCK inhibitor

Seeding density: $1.0 \times 10^4$ cells/cm$^2$
Replacement of medium: 0.5 mL/well, once every two days
Added reagent: Methylglyoxal Solution (manufactured by Sigma-Aldrich)
Methylglyoxal concentration: 0,1, 5, 10, 15, 20 mM
Addition of MG: MG was added after the medium was replaced on the 3rd day of culture (Day 3).
MG treatment time: 24 hours

The proliferation of cells in the cell aggregate (i.e., an increase in the diameter of the cell aggregate) could hardly be observed on Day 3 (before the addition of MG). This cell aggregate was treated with MG. Consequently, the periphery of the cell aggregate was found to expand slightly. In other words, the above treatment and culture had the effect of promoting cell proliferation.

Thus, the results indicate that the above treatment can restore the proliferative capacity of the HepG2 cell aggregate, as in the case of the iPS cell aggregate and the MSC cell aggregate.

## Claims

1. A method for promoting proliferation of cells,

   the method comprising:
   adding a precursor of advanced glycation end products (AGEs) to a culture medium containing a cell aggregate; and
   culturing the cell aggregate in the culture medium to which the precursor of AGEs has been added

2. The method according to claim 1, comprising:
   adding a Rho-kinase inhibitor (ROCK inhibitor) to the culture medium at the same time as or after the addition of the precursor of AGEs.

3. The method according to claim 1 or 2, wherein the cells are stem cells.

4. The method according to any one of claims 1 to 3, wherein the precursor of AGEs is at least one of an $\alpha$-dicarbonyl compound or an $\alpha$-hydroxy aldehyde compound

5. The method according to claim 4, wherein the $\alpha$-dicarbonyl compound is selected from the group consisting of methylglyoxal, glyoxal, 3-Deoxyglucosone, malondialdehyde, butanedione, 1,2-Cyclohexanedione, phenylglyoxal,

4-Fluorophenylglyoxal, 4-Nitrophenylglyoxal, 4-Hydroxyphenylglyoxal, and combinations thereof

**6.** The method according to claim 4 or 5, wherein the α-hydroxy aldehyde compound is selected from the group consisting of glyceraldehyde, glycolaldehyde, lactaldehyde, 3-Hydroxybutyraldehyde, and combinations thereof

**7.** A method for preparing a cell aggregate,

the method comprising:
adding a precursor of advanced glycation end products (AGEs) to a culture medium containing a cell aggregate; and
culturing the cell aggregate in the culture medium to which the precursor of AGEs has been added

**8.** The method according to claim 7, comprising:
adding a Rho-kinase inhibitor (ROCK inhibitor) to the culture medium at the same time as or after the addition of the precursor of AGEs.

**9.** A method for unraveling an extracellular matrix shell (ECM shell) of a cell aggregate, the method comprising:

culturing cells to form a cell aggregate; and
adding a precursor of advanced glycation end products (AGEs) to a culture medium containing the cell aggregate.

**10.** A cell aggregate obtained by the method according to any one of claims 7 to 9.

**11.** An anti-fibrillating agent for a cell aggregate,
the anti-fibrillating agent comprising a precursor of advanced glycation end products (AGEs).

**12.** A kit for culturing a cell aggregate,
the kit comprising:

a precursor of advanced glycation end products (AGEs); and
a Rho-kinase inhibitor (ROCK inhibitor).

**13.** A method for preparing a cell aggregate,
the method comprising:

adding an α-dicarbonyl compound to a culture medium containing a cell aggregate, and
culturing the cell aggregate in the culture medium to which the α-dicarbonyl compound has been added

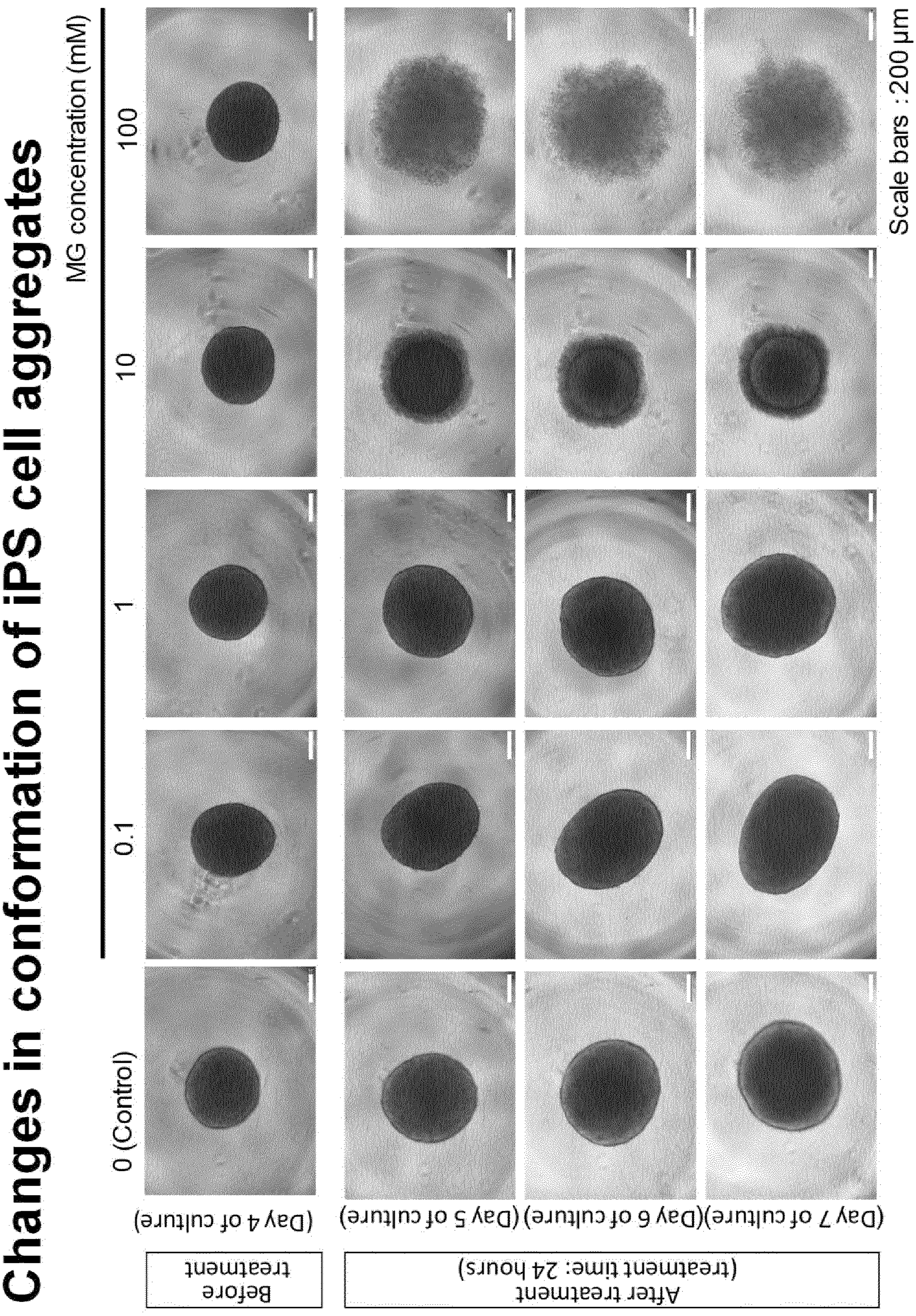
Changes in conformation of iPS cell aggregates
MG concentration (mM)
0 (Control)
0.1
1
10
100
Before treatment
(Day 4 of culture)
After treatment
(treatment time: 24 hours)
(Day 5 of culture)
(Day 6 of culture)
(Day 7 of culture)
Scale bars :200 μm

FIG. 1A

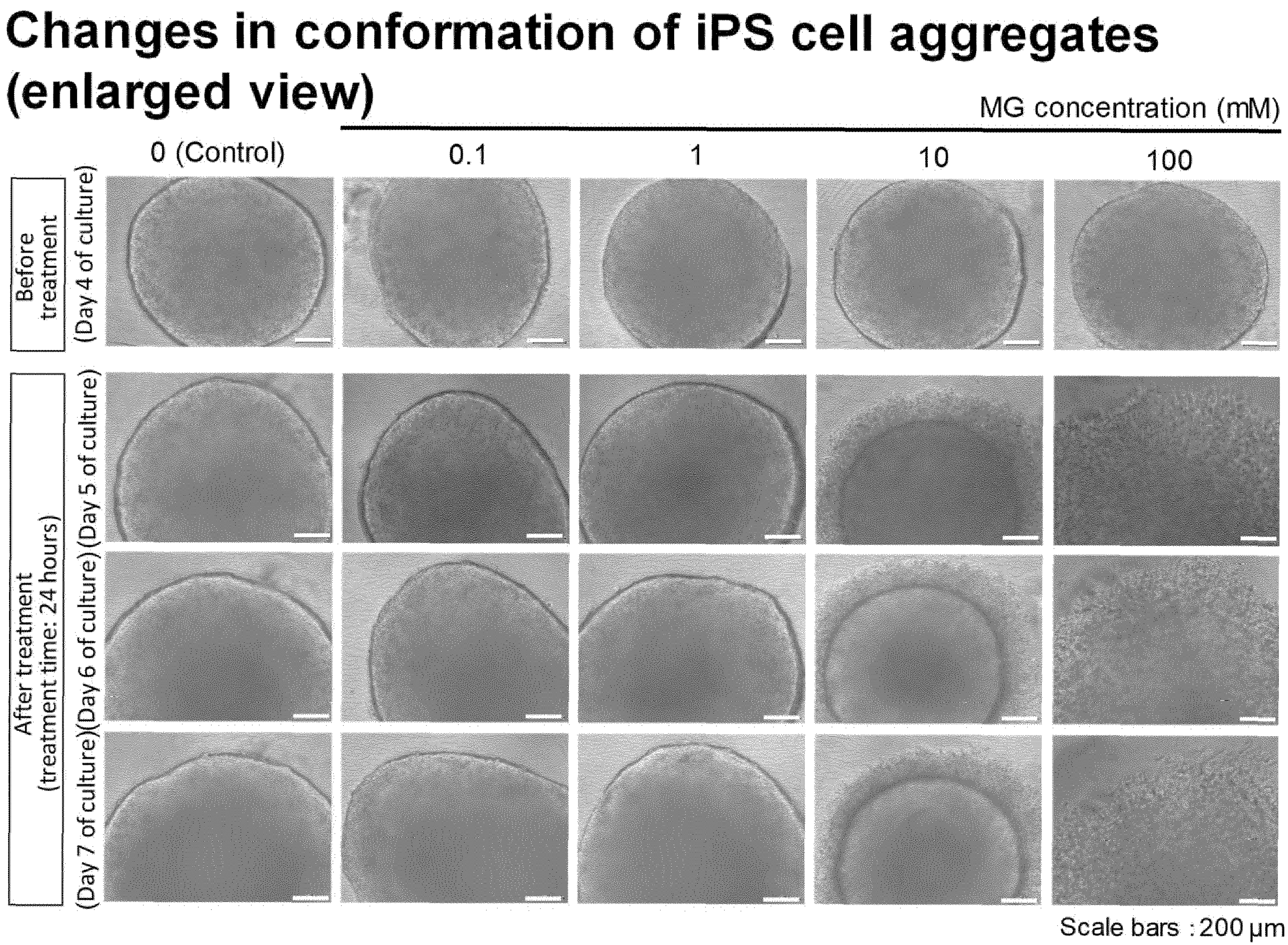
Changes in conformation of iPS cell aggregates (enlarged view)
MG concentration (mM)
0 (Control)
0.1
1
10
100
Before treatment
(Day 4 of culture)
After treatment
(treatment time: 24 hours)
(Day 5 of culture)
(Day 6 of culture)
(Day 7 of culture)
Scale bars : 200 µm

FIG. 1B

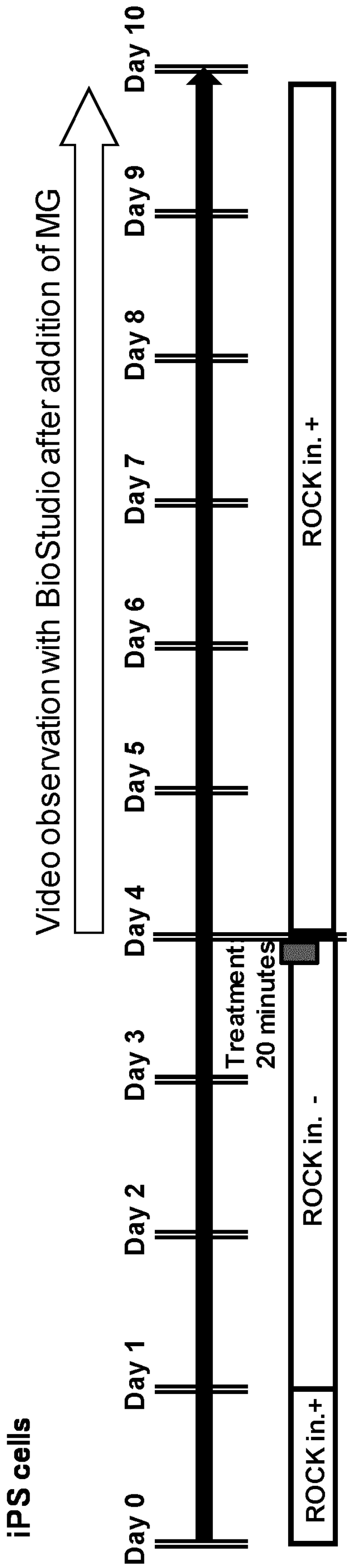
iPS cells
Video observation with BioStudio after addition of MG
Day 0
Day 1
Day 2
Day 3
Day 4
Day 5
Day 6
Day 7
Day 8
Day 9
Day 10
Treatment
20 minutes
ROCK in.+
ROCK in. -
ROCK in. +

FIG. 2

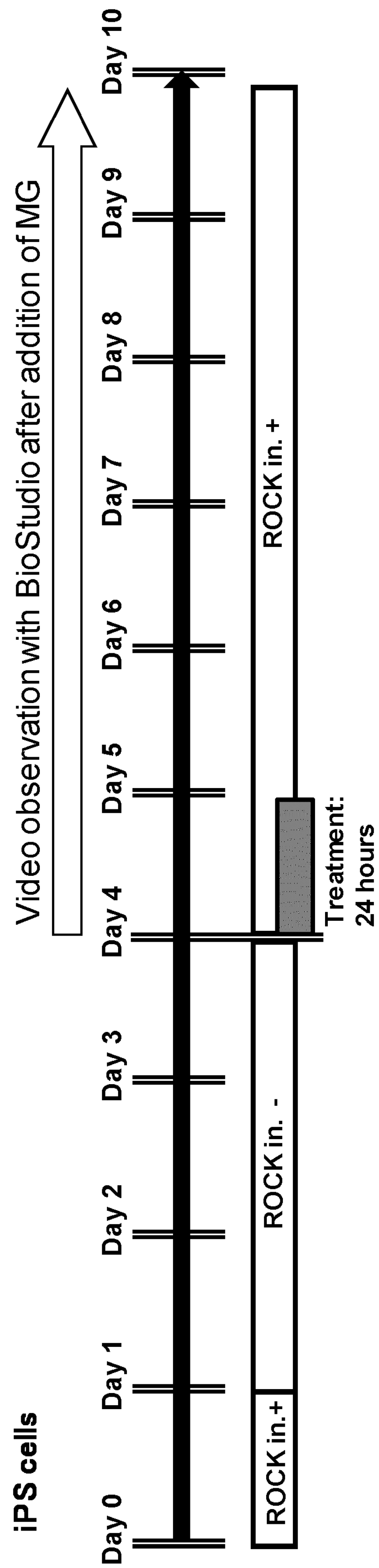
iPS cells
Video observation with BioStudio after addition of MG
Day 0
Day 1
Day 2
Day 3
Day 4
Day 5
Day 6
Day 7
Day 8
Day 9
Day 10
Treatment:
24 hours
ROCK in.+
ROCK in. -
ROCK in. +

FIG. 3

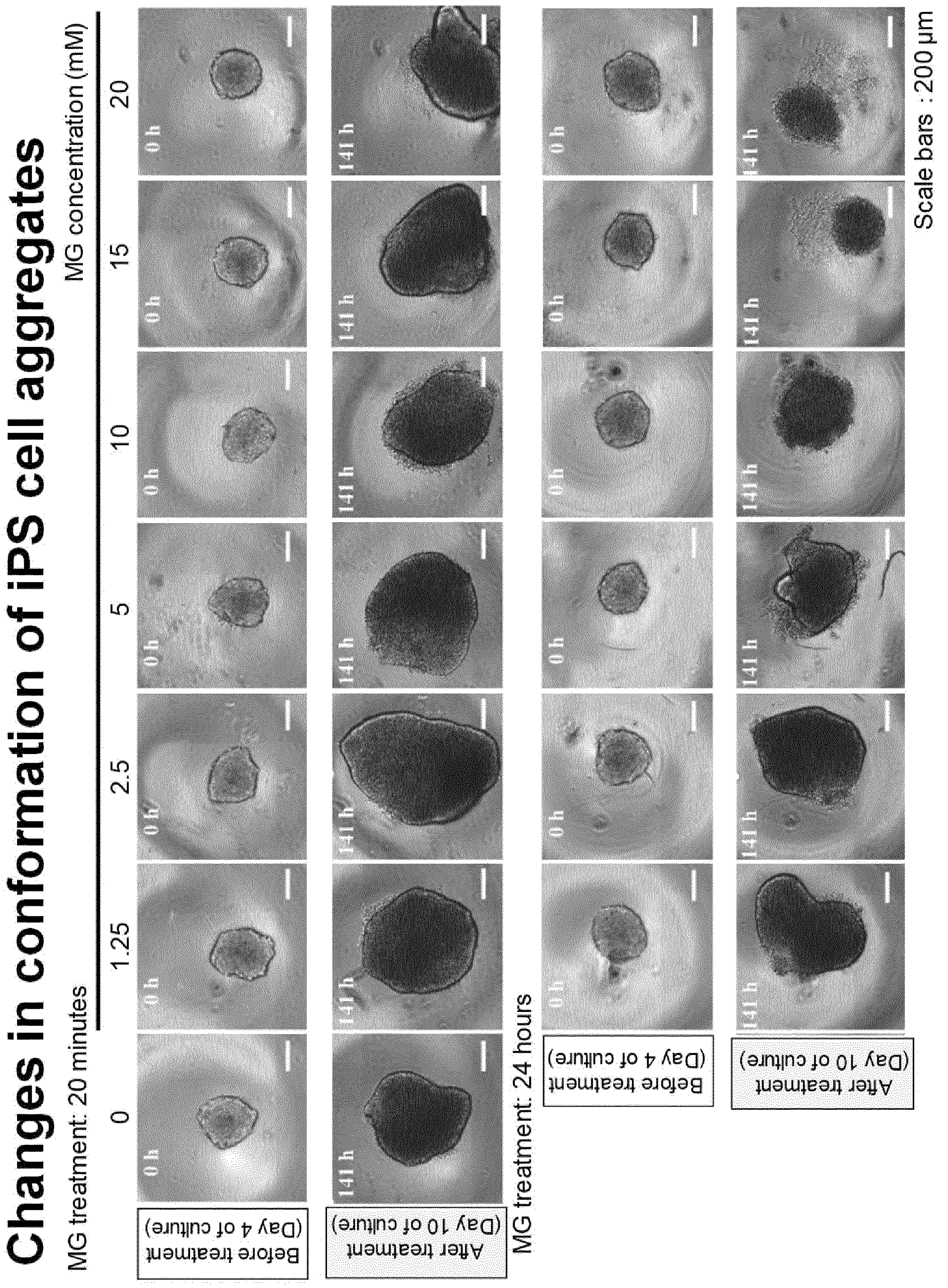
Changes in conformation of iPS cell aggregates
MG treatment: 20 minutes
MG concentration (mM)
0
1.25
2.5
5
10
15
20
Before treatment (Day 4 of culture)
After treatment (Day 10 of culture)
MG treatment: 24 hours
Before treatment (Day 4 of culture)
After treatment (Day 10 of culture)
0 h
141 h
Scale bars : 200 μm

FIG. 4

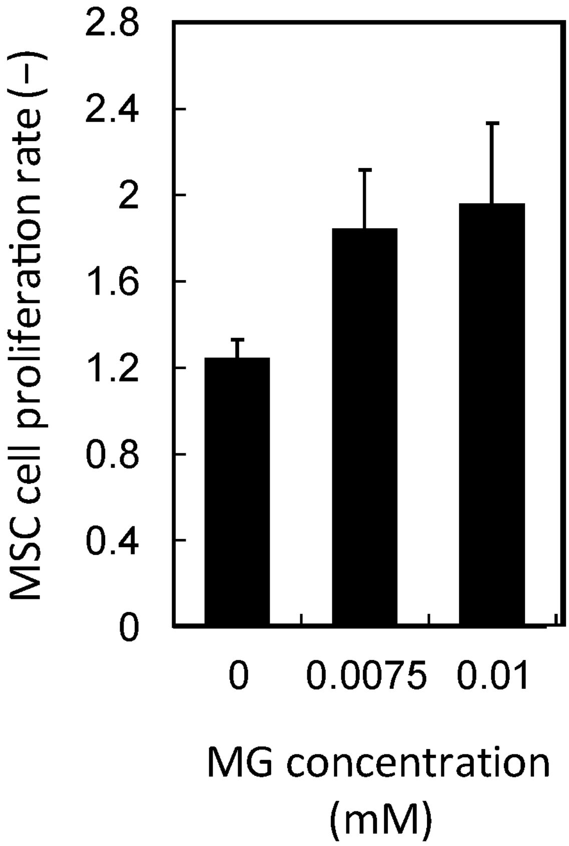
MSC cell proliferation rate (−)
2.8
2.4
2
1.6
1.2
0.8
0.4
0
0
0.0075
0.01
MG concentration (mM)

FIG. 5

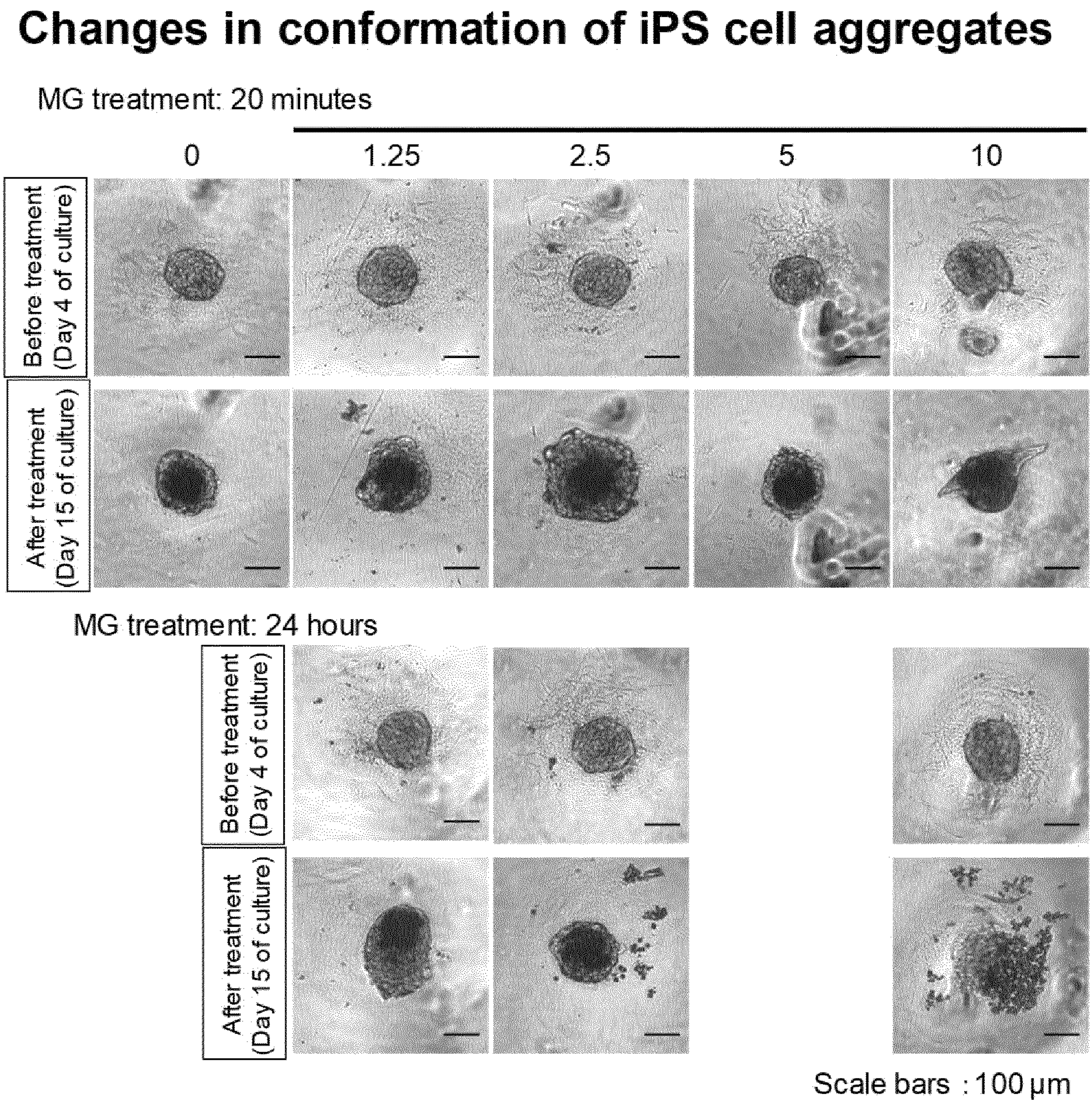
Changes in conformation of iPS cell aggregates
MG treatment: 20 minutes
0
1.25
2.5
5
10
Before treatment (Day 4 of culture)
After treatment (Day 15 of culture)
MG treatment: 24 hours
Before treatment (Day 4 of culture)
After treatment (Day 15 of culture)
Scale bars : 100 μm

FIG. 6

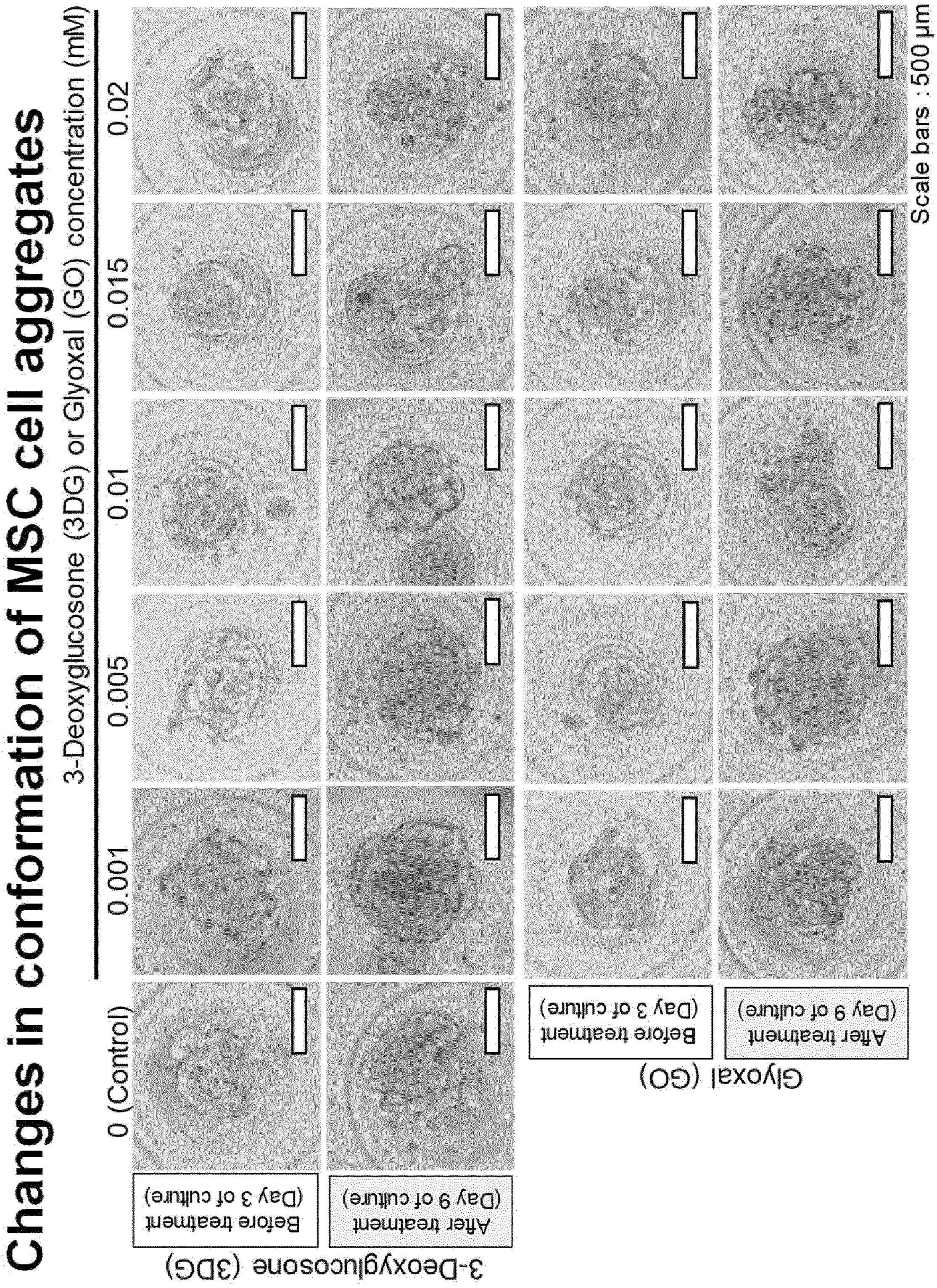
Changes in conformation of MSC cell aggregates
3-Deoxyglucosone (3DG) or Glyoxal (GO) concentration (mM)
0 (Control)
0.001
0.005
0.01
0.015
0.02
3-Deoxyglucosone (3DG)
Before treatment (Day 3 of culture)
After treatment (Day 9 of culture)
Glyoxal (GO)
Before treatment (Day 3 of culture)
After treatment (Day 9 of culture)
Scale bars : 500 μm

FIG. 7

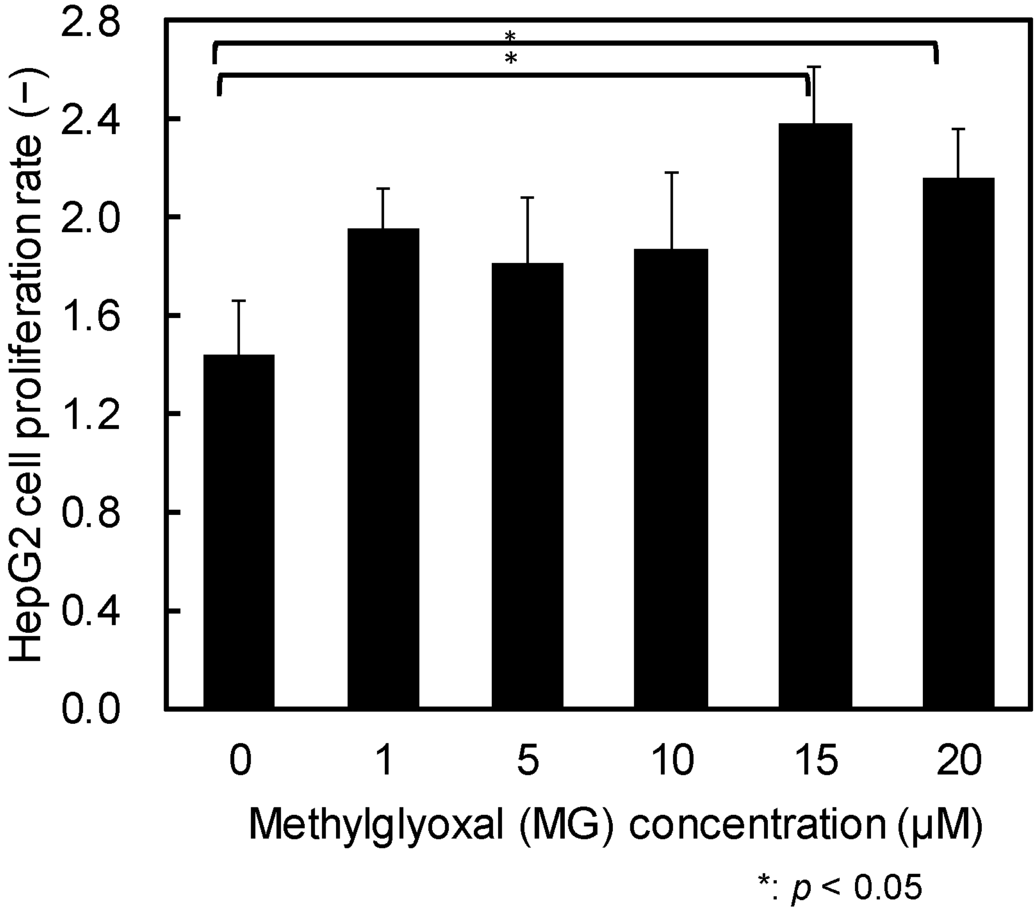
2.8
2.4
2.0
1.6
1.2
0.8
0.4
0.0
HepG2 cell proliferation rate (−)
*
*
0
1
5
10
15
20
Methylglyoxal (MG) concentration (μM)
*: p < 0.05

FIG. 8

**INTERNATIONAL SEARCH REPORT**

International application No. PCT/JP2020/027111

A. CLASSIFICATION OF SUBJECT MATTER
C12N 5/071(2010.01)i; C12N 5/10(2006.01)i
FI: C12N5/071; C12N5/10

According to International Patent Classification (IPC) or to both national classification and IPC

B. FIELDS SEARCHED

Minimum documentation searched (classification system followed by classification symbols)
C12N5/071; C12N5/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | ROEHLECKE, C. et al., "Resistance of L132 lung cell clusters to glyoxal-induced apoptosis", Histochem. Cell Biol., 2000, vol. 114, 283-292 abstract, Introduction, Results, "Glyoxal causes disintegration of spheroids", "Disintegration of spheroids is associated with decreased expression of adhesion molecules", Discussion | 7, 3-11, 13<br>1-13 |
| Y | WO 2016/121840 A1 (THE UNIVERSITY OF TOKYO) 04.08.2016 (2016-08-04) claims, examples | 1-13 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 August 2020 (25.08.2020) | 15 September 2020 (15.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/027111

| Patent Documents referred in the Report | Publication Date | Patent Family | Publication Date |
|---|---|---|---|
| WO 2016/121840 A1 | 04 Aug. 2016 | US 2018/0023057 A1<br>claims, examples<br>EP 3252154 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015033558 A
- WO 2014017513 A